Europäisches Patentamt

European Patent Office

Office européen des brevets

(11) Veröffentlichungsnummer: **0 029 892**
**B1**

(12) **EUROPÄISCHE PATENTSCHRIFT**

(45) Veröffentlichungstag der Patentschrift:
**02.11.83**

(21) Anmeldenummer: **80105867.8**

(22) Anmeldetag: **27.09.80**

(51) Int. Cl.³: **C 23 F 11/12, C 10 M 1/08**

(54) **Verwendung von Alkali- oder Aminsalzen eines Gemisches aus 2- und 3-Alkyladipinsäuren als Korrosionsinhibitor.**

(30) Priorität: **01.12.79 DE 2948503**

(43) Veröffentlichungstag der Anmeldung:
**10.06.81 Patentblatt 81/23**

(45) Bekanntmachung des Hinweises auf die Patenterteilung:
**02.11.83 Patentblatt 83/44**

(84) Benannte Vertragsstaaten:
**FR GB NL**

(56) Entgegenhaltungen:
**DE - B - 1 285 835**
**FR - A - 1 105 891**
**US - A - 2 300 955**
**US - A - 2 426 496**
**US - A - 2 726 215**
**US - A - 3 696 048**

(73) Patentinhaber: **CHEMISCHE WERKE HÜLS AG,**
**Postfach 1320, D-4370 Marl 1 (DE)**

(72) Erfinder: **Keil, Herwalt, Dr., verstorben (DE)**
Erfinder: **Alfs, Helmut, Bitterfelder Strasse 47,**
**D-4370 Marl (DE)**
Erfinder: **Schulze, Klaus, Dr., Burgstrasse 163,**
**D-4358 Haltern (DE)**

## Verwendung von Alkali- oder Aminsalzen eines Gemisches aus 2- und 3-Alkyladipinsäuren als Korrosionsinhibitor

Aus der US-PS 2 726 215 ist es bereits bekannt, Alkalisalze der Azelain- oder Sebazinsäuren als Korrosionsinhibitoren für wässrige Lösungen einzusetzen, während die DE-OS 1 444 903 die Verwendung von Alkanolaminsalzen von Azelain- und Sebazinsäure in wasserhaltigen Bohr- und Schneidflüssigkeiten auf Polyoxialkylen-Basis beschreibt. Die Korrosionsschutzeigenschaften von Salzen der Azelain- und Sebazinsäure sind jedoch nicht optimal und lassen noch Raum zu Verbesserungen, wie aus den Tabellen 1 und 3 hervorgeht.

Durch die DE-PS 917 027 gehört es bereits zum Stand der Technik, Kohlenwasserstoffölen als Rostinhibitor Alkylbernsteinsäure zuzusetzen, während in der DE-AS 1 063 311 der Zusatz von Alkyladipinsäuren mit kurzer Seitenkette, wie Methyladipinsäure, beschrieben ist.

Alkylbernsteinsäuren zeigen in wasserhaltigen Medien den Nachteil, mit den Härtebildnern des Wassers unlösliche Erdalkalisalze zu bilden und somit auszufallen, während kurzkettige Alkyladipinsäuren selbst als Aminsalze noch einen unzureichenden Rostschutz ergeben (vgl. Tabelle 1).

Aus der DE-AS 1 444 904 und der DE-OS 1 644 907 ist es bekannt, Schmierölen zur Reinigung bzw. zum Verhindern von Schlammbildung Aminsalze von substituierten Bernsteinsäuren zuzusetzen. Diese Aminsalze tragen Substituenten mit einer C-Zahl ≥ 30. Zum einen ist diesen Literaturstellen keine Lehre zu entnehmen, wie Metallkorrosionen in wasserhaltigen Systemen zu verhindern sind, zum anderen zeigen die betreffenden Aminsalze aufgrund der hohen C-Zahl ihrer Substituenten keine ausreichende Wasserlöslichkeit.

In der Praxis werden aus wirtschaftlichen Gründen für eine einfache Metallbearbeitung, z.B. zum Schleifen und Glätten, immer noch wasserhaltige Flüssigkeiten verwendet, die Alkanolamine und Natriumnitrit enthalten. Es hat sich jedoch gezeigt, dass aus Nitrit und Alkanolaminen carcinogene Nitrosamine gebildet werden. Als rosthemmende Zusätze zu Metallbearbeitungsmitteln in wasserhaltigen Systemen werden weiterhin vielfach zwei Handelsprodukte eingesetzt. Handelsprodukt A ist das Triethanolaminsalz einer Arylsulfonamidoalkylencarbonsäure (DE-PS 1 298 672), Handelsprodukt B eine Mischung aus Fettsäurediethanolamid und Fettsäurediethanolaminsalzen. Beide Handelsprodukte zeigen laut Tabelle 1 eine Rostschutzwirkung, welche vor allem im geringen Konzentrationsbereich noch der Verbesserung bedarf.

Diese Nachteile des Standes der Technik werden vermieden durch Verwendung von Dialkalisalzen oder Disalzen wasserlöslicher aliphatischer Amine eines Gemisches aus 2- und 3-Alkyladipinsäuren, deren Alkylrest verzweigt oder unverzweigt ist und 6 bis 12 Kohlenstoffatome enthält, als Korrosionsinhibitor für Bohr-, Schneid- und Schleifmittel, Hydraulikflüssigkeiten und Gefrierschutzmittel, welche Wasser als Hauptbestandteil enthalten.

Diese Alkali- oder Aminsalze werden in Bohr-, Schneid- und Schleifmitteln in Konzentrationen von 0,5 bis 3 Gewichtsprozent, bezogen auf das gesamte gebrauchsfähige Bohr-, Schneid- oder Schleifmittel, eingesetzt.

In Hydraulikflüssigkeiten werden diese Alkalioder Aminsalze in Konzentrationen von 1 bis 5 Gewichtsprozent, bezogen auf die gesamte Hydraulikflüssigkeit, verwendet.

In Gefrierschutzmitteln werden die Alkali- oder Aminsalze in Konzentrationen von 0,6 bis 1,7 Gewichtsprozent, bezogen auf das gesamte Gefrierschutzmittel, eingesetzt.

Werden die Alkali- oder Aminsalze gemeinsam mit anderen üblichen Korrosionsinhibitoren in Gefrierschutzmitteln eingesetzt, so genügt eine Konzentration von 0,6 bis 1,0 Gewichtsprozent, bezogen auf das gesamte Gefrierschutzmittelkonzentrat.

Die Gemische aus 2- und 3-Alkyladipinsäuren, deren Alkali- oder Aminsalze erfindungsgemäss zu verwenden sind, lassen sich herstellen durch die bekannte Alkylierung von Phenol mit einem 6 bis 12 Kohlenstoffatome enthaltenden Olefin in Gegenwart von Säuren oder Lewis-Säuren oder sauren Ionenaustauschern, katalytische Hydrierung des erhaltenen Alkylphenols in bekannter Weise zum Alkylcyclohexanol und Oxidation mit Salpetersäure in gleichfalls üblicher Weise.

Eine besonders günstige Methode der Oxidation von Alkylcyclohexanol mit Salpetersäure ist in der DE-PS 1 643 854 beschrieben.

Die Alkylphenolgemische, welche als Ausgangsstoffe zur Herstellung der Gemische aus 2- und 3-Alkyladipinsäuren in Frage kommen, lassen sich durch Anlagerung von Olefinen, wie Hexen, Octen, Diisobutylen, Tripropylen, Tetrapropylen oder Dodecen an Phenol herstellen. Aus Diisobutylen und Phenol erhält man ein Gemisch aus vorwiegend p- und wenig o-Isooctylphenol; aus Tripropylen und Phenol ein Gemisch aus vorwiegend p- und wenig o-Isononylphenol. Bei der Anlagerung von unverzweigten Olefinen an Phenol erhält man dagegen Gemische aus dem o- und p-Isomeren, in welchen das o-Isomere überwiegt. Eine besonders günstige Arbeitsweise zur Herstellung der Alkylphenole ist in J. Klein, H. Widdecke, Chem.-Ing.-Techn. 51 (1979) 6, 560 beschrieben.

Wenn im folgenden von Alkyladipinsäure(n) gesprochen wird, so soll stets ein Gemisch aus 2- und 3-Alkyladipinsäuren gemeint sein, wie es z.B. durch Anlagerung von Olefinen an Phenol, Hydrierung der Alkylphenole zu Alkylcyclohexanolen und nachfolgende Oxidation, z.B. mit Salpetersäure, erhalten wird.

Als Alkyladipinsäuren, deren Alkali- oder Aminsalze erfindungsgemäss zu verwenden sind, lassen sich z.B. heranziehen: n-Hexyladipinsäure, n-Octyladipinsäure, n-Decyladipinsäure, n-Dodecyl-

adipinsäure, Isooctyladipinsäure, Isononyladipinsäure, Isododecyladipinsäure, n-Tetradecyladipinsäure, Isopentadecyladipinsäure, n-Hexadecyladipinsäure.

Zur Herstellung der erfindungsgemäss einzusetzenden Aminsalze der Alkyladipinsäuren eignen sich wasserlösliche aliphatische Amine, Diamine, Alkanolamine sowie Polyalkylenpolyamine.

Beispiele sind Methylamin, Ethylamin, n-Propylamin, n-Butylamin, n-Pentylamin, Dimethylamin, Diethylamin, Di-n-propylamin, Di-n-butylamin, Isopropylamin, Isobutylamin, Isopentylamin, Diisopropylamin, Diisobutylamin, Alkanolamine wie Ethanolamin, Diethanolamin, Triethanolamin, Isopropanolamin, Diisopropanolamin, Triisopropanolamin, Alkylalkanolamine wie Methyldiethanolamin, Ethyldiethanolamin, Propyldiethanolamin, Butyldiethanolamin, Pentyldiethanolamin, Hexyldiethanolamin, Dimethylethanolamin, Diethylethanolamin, Dipropylethanolamin, Dibutylethanolamin, Dipentylethanolamin, Diamine wie N-ethylpropandiamin, N-Diethylpropandiamin, N-propylpropandiamin, Morpholylpropylamin, Polyalkylenpolyamine wie Ethylendiamin, Diethylentriamin, Triethylentetramin, Tetraethylenpentamin, sowie deren Oxethylierungsprodukte mit bis zu 10 Oxethylgruppen.

Als Aminsalze zum erfindungsgemässen Einsatz bevorzugt geeignet sind Triethanol- und Triisopropanolaminsalze der Isooctyl- und Isononyladipinsäure. Es eignen sich jedoch auch die Triethanolamin- und Triisopropanolaminsalze der n-Hexyladipinsäure, der n-Octyladipinsäure, der n-Decyladipinsäure, der n-Dodecyladipinsäure und der Isododecyladipinsäure.

Die erfindungsgemäss zu verwendenden Aminsalze werden zweckmässigerweise hergestellt, indem man das Amin mit der Alkyladipinsäure zusammengibt und während eines Zeitraumes von 5 bis 15 Minuten unter Rühren auf 60 bis 90 °C erwärmt.

Es ist auch möglich, die erhaltenen Aminsalze für einen Zeitraum von 2 bis 6 Stunden auf Temperaturen von 120 bis 250 °C zu erhitzen und unter Abspaltung eines Mols Wasser das Säureamid herzustellen. Diese Reaktion lässt sich auch mit Hilfe eines Schleppmittels wie Toluol oder Xylol ausführen. Die so erhaltenen Säureamide lassen sich gleichfalls mit Erfolg als Korrosionsinhibitoren im hier beschriebenen Umfang einsetzen.

Erfindungsgemäss zu verwendende Alkalisalze der Alkyladipinsäuren sind Na- und K-Salze.

Als Medien, in denen die beschriebenen Salze der Alkyladipinsäuren erfindungsgemäss angewendet werden, kommen z.B. folgende bekannte Mittel in Frage:

1) Bohr-, Schneid- und Schleifmittel, bestehend aus:
   a) 90 bis 98 Gewichtsprozent Wasser,
      10 bis 2 Gewichtsprozent Glykolen, Polyglykolen, Polyglykolether oder wasserlöslichen Polymeren aus Ethylen- und Propylenoxid oder Homo- bzw. Copolymeren aus Acryl-, Methacrylsäure oder Maleinsäureanhydrid und Olefinen und untergeordneten Mengen an Buntmetallinhibitoren, oder
   b) 90 bis 98 Gewichtsprozent Wasser,
      7 bis 2 Gewichtsprozent Mineralöl,
      3 bis 1 Gewichtsprozent Emulgatoren.
      Diese Mittel kommen als Emulsionen zum Einsatz.

2) Hydraulikflüssigkeiten, bestehend aus:
   a) 40 bis 50 Gewichtsprozent Wasser,
      20 bis 25 Gewichtsprozent eines Verdickungsmittels, z.B. eines Mischaddukts aus Ethylenoxid und Propylenoxid,
      35 bis 20 Gewichtsprozent eines Glykols,
      10 bis 5 Gewichtsprozent üblicher Zusätze, wie Inhibitoren, Alkanolamine, Dampfphasenkorrosionsschutzmittel,
   b) Öl-in-Wasser-Emulsionen mit üblichen Zusätzen,

3) Gefrierschutzmittel, bestehend aus:
      25 bis 40 Gewichtsprozent inhibiertem Glykol,
      Rest zu 100 Gewichtsprozent Wasser. Als Inhibitorbestandteile kommen z.B. zur Anwendung:
      Na-Benzoat, Borax, Na-Nitrit, Na-Nitrat, Na-Metasilikat, Benzotriazol, ε-Aminopentylimidazolin, Natriumcarbonat. Ferner können bekannte Entschäumer angewandt werden.

Die fertigen Einstellungen sollten einen pH-Wert zwischen 7,0 und 9,0, vorzugsweise 7,2 und 8,0, haben.

Die erfindungsgemäss zu verwendenden Alkalisalze von Alkyladipinsäuren finden ihren Einsatz insbesondere in Bremsflüssigkeiten und in Gefrierschutzmitteln.

In den Bremsflüssigkeiten des Standes der Technik kann das Borax beispielsweise durch das Kaliumsalz von Alkyladipinsäuren substituiert werden, wobei mindestens gleich gute Korrosionsschutzergebnisse und verbesserte Reibverschleissschutzeigenschaften erzielt werden.

In Gefrierschutzmitteln können die Alkalisalze von Alkyladipinsäuren in gängigen Rezepturen Natriumbenzoat ersetzen, wodurch verbesserte Korrosionsschutzwerte erhalten werden.

Übliche Korrosionsinhibitoren, welche zusammen mit den erfindungsgemäss zu verwendenden Salzen von Alkyladipinsäuren eingesetzt werden können, sind beispielsweise: Na-Nitrit, Borax, Na-Nitrat, Na-Metasilikat und ein Buntmetallinhibitor, wie z.B. ε-Aminopentylimidazolin, Benzotriazol.

Zur weiteren Erläuterung der Erfindung und zum Nachweis des erzielbaren technischen Fortschritts dienen die folgenden Beispiele:

Beispiel 1

Herstellung von Isooctyladipinsäure

(Dieses Herstellungsbeispiel kann sinngemäss für alle erfindungsgemäss einzusetzenden Alkyladipinsäuren gelten).

340 kg Diisobuten und

570 kg Phenol werden gemischt, auf ca. 70 °C vorgewärmt und durch zwei hintereinandergeschaltete, mit jeweils
120 kg Katalysator gefüllte Reaktoren gepumpt
(Detailbeschreibungen in der DE-PS
2 346 273).

Das Rohalkylat enthielt ca. 63% Octylphenol.

Nach destillativer Aufarbeitung erhält man ein
Octylphenol mit folgenden Daten:

| | |
|---|---|
| Gehalt an Phenol | ≤ 0,2 Gew.-% |
| Gehalt an Octylphenol | ≥95 Gew.-% |
| Rest (überwiegend Dialkylphenol) | ≤ 5 Gew.-% |

Das Octylphenol besteht zu ca. 98% aus para-
und zu ca. 2% aus ortho-Isomeren.

Die Hydrierung erfolgt unter Standardbedingungen bei 160°C unter 1 at Druck in Gegenwart von
Pd auf $Al_2O_3$ (Engelhardt-Kontakt).

Das durch Destillation erhaltene Octylcyclohexanol besitzt einen hohen Reinheitsgrad und
beinhaltet folgende Daten:

| | |
|---|---|
| Farbe APHA | ~5 |
| d 20/4 | 0,9254 |
| Phenol | <0,01% |
| OH-Zahl | 261,4 |
| $Kp_{27}$ mbar | 161,5–167,5 |

Oxidation:
Apparatur: 4-Liter-Rührkolben, Tropftrichter, Temperaturmessung, Kühlbad.

In 1300 g Salpetersäure «40» Be werden bei 60
bis 65°C innerhalb 3 Stunden 424 g Octylcyclohexanol eingetropft. Anschliessend bei 60°C 1
Stunde nachgerührt, der Kolbeninhalt über Nacht
stehen gelassen, abgesaugt und mit Eiswasser
$HNO_3$-frei gewaschen.

Die getrocknete Säure (31 kg) hatte eine Säurezahl von 421.

In Tabelle 1, 2 und 3 werden bekannte Korrosionsinhibitoren auf Basis von Mono- und Dicarbonsäuren gegenübergestellt und in ihrer Korrosionsschutzwirkung miteinander verglichen. In
den angegebenen Rezepturen wurde die Gesamtmenge an Inhibitor, also Säure und Amine, jeweils
etwa gleich belassen und das Amin/Säure-Ver-
hältnis so eingestellt, dass der pH-Wert einer
5%igen, wässrigen Lösung etwa gleichlaufend 7,5
betrug.

Die Ausprüfung auf Korrosionsschutzwirkung
erfolgte nach DIN 51 360, Teil 1 und Teil 2.

In der Ausführungsform Teil 1 werden Stahlfrässpäne auf eine gereinigte Prüfplatte aus Gusseisen gebracht und mit dem wassergemischten
Kühlschmierstoff benetzt. Nach einer Verweilzeit
von 24 Stunden in der Prüfkammer bei Raumtemperatur und einer relativen Luftfeuchte von 50 bis
60% werden die Stahlspäne und die ölfreie Oberfläche der Prüfplatte auf Korrosion untersucht.

Gemäss Verfahren Teil 2 werden Graugussspäne GG 30 auf einem Rundfilter mit der frisch
zubereiteten, wässrigen Korrosionsschutzlösung
benetzt, 2 Stunden lang in einer geschlossenen
Petrischale bei Raumtemperatur belassen und anschliessend das Rundfilter auf Korrosionsabzeichnungen visuell beurteilt.

Die Resultate lassen erkennen, dass Benzoesäure, deren alkylierte Derivate, Ölsäuren,
Adipinsäure bzw. kurzkettig alkylierte Adipinsäuren im Konzentrationsbereich 0,5 bis 2 Gewichtsprozent auf Eisen keine oder nur eine geringe Rostschutzwirkung zeigen. Auf Vergleichsversuche mit Salzen von Alkylbernsteinsäuren wurde
verzichtet, da diese Säuren bei Verwendung von
Wasser 8° dGH mit den Härtebildnern Niederschläge bildeten. Alkyladipinsäuren ab einer Kettenlänge von 6 C-Atomen zeigen in Verdünnungen
ab 1% sehr guten Korrosionsschutz. In geringen
Konzentrationen erweist sich diese Rostschutzwirkung deutlich besser als im Vergleich zu den
gleichzeitig ausgeprüften Azelain- und Sebazinsäuren. Auch bei den Alkalisalzen dieser Säuren,
die als reine, trockene Salze zum Ansetzen der
Lösungen verwendet wurden, ergeben die Alkalisalze der Alkyladipinsäuren in geringen Konzentrationen bis 1 Gewichtsprozent eine ebenfalls
deutlich erkennbare Verbesserung der Korrosionsschutzwirkung, verglichen mit den Lösungen der Alkalisalze der Azelain- und Sebazinsäure.

Ein Vergleich mit handelsüblichen Korrosionsschutzmitteln, Handelsprodukt A (alkylsulfonamidoalkylencarbonsaures Triethanolamin) und
Handelsprodukt B (Mischung aus Fettsäurediethanolamid und Fettsäureethanolaminsalzen),
ergab eine deutlich bessere Rostschutzwirkung
der erfindungsgemässen Zusätze. Im Vergleich zu
Handelsprodukt A zeigte sich eine Überlegenheit
der erfindungsgemässen Korrosionsschutzmittel
in geringen Konzentrationsbereichen unter 2 Gewichtsprozent.

Bewertung des Korrosionsgrades nach DIN 51 360
Teil 1 in Tabelle 1 bis 3
0 = keine Korrosion,
1 = Spuren einer Korrosion,
2 = leichte Korrosion (korrodierte Fläche 10%),
3 = mässige Korrosion (korrodierte Fläche 10 bis
25%),
4 = verstärkte Korrosion (korrodierte Fläche 25
bis 50%),
5 = starke Korrosion (korrodierte Fläche 50 bis
75%),
6 = sehr starke Korrosion (über 75% korrodierte
Fläche).

Korrosionsgrade der Korrosionsabzeichnungen
auf den Rundfilter nach DIN 51 360, Teil 2
0 = keine Korrosion, unveränderte Oberfläche
des Rundfilters,
1 = Spuren von Korrosion, höchstens 3 Rostabzeichnungen,
2 = leichte Korrosion, nicht mehr als 1% der
Oberfläche verfärbt,
3 = mässige Korrosion, bis zu 5% der Oberfläche
verfärbt,
4 = starke Korrosion, über 5% der Oberfläche
rostig verfärbt.

Beispiel 2
Nach üblicher Methode wurde ein emulgierbares Mineralöl aus einem Spindelölraffinat
20 mm²/s bei 50°C unter Verwendung eines Natriumpetrolsulfonats vom Molgewicht 450 und eines Ölsäure-Triethanolaminsalzes hergestellt. Die wässrige Emulsion zeigte in einer Konzentration von 5 Vol.-% nach DIN 51 360, Teil 2, eben noch einen ausreichenden Korrosionsschutz. Nach Zugabe von 4 Gewichtsprozent einer Mischung gemäss Beispiel 10, Tabelle 2, oder 4 Gewichtsprozent einer 40%igen Lösung des Kaliumsalzes der Isononyladipinsäure in Butyldiglykol/Wasser zum emulgierbaren Mineralölkonzentrat, war mit einer 2%igen Emulsion noch ein guter Korrosionsschutz zu erreichen. Für denselben Rostschutz war die Zugabe von 8 Gewichtsprozent Handelsprodukt B oder 7 Gewichtsprozent des mineralölhaltigen Standardproduktes Handelsprodukt A erforderlich, was den überraschenden Effekt der erfindungsgemässen Korrosionsinhibitoren beweist.

Auch bei Ersatz des Emulgators auf Basis eines Natriumpetrolsulfonats durch geeignete nichtionogene Emulgatoren, beispielsweise des Typs oxethyliertes Nonylphenol oder eines Ölsäureoxethylats werden im Korrosionsschutz bei Zugabe des Kaliumsalzes von Alkyladipinsäuren gleichlaufende Befunde beobachtet.

Beispiel 3

Zur Herstellung inhibierter Glykole zur Verwendung als Gefrierschutzmittel für Automobilkühlsysteme wurden nach ASTM D 1384-70 folgende Inhibitormischungen ausgeprüft. Die Angaben in Tabelle 4 verstehen sich in Gewichtsprozent:

Tabelle 4

|  | 1 | 2 | 3 | 4 | 5 | 6 | 7 |
|---|---|---|---|---|---|---|---|
| Natriumbenzoat | 5,00 | – | 4,00 | – | 2,50 | – | – |
| Natriumsalz der 2-Isooctyladipinsäure | – | 5,00 | – | 4,00 | – | 2,50 | 2,50 |
| Borax + 10 Wasser | – | – | 1,00 | – | 1,40 | 1,40 | 1,40 |
| Natriumnitrit | – | – | 0,45 | 0,40 | 0,25 | 0,25 | 0,25 |
| Natriumnitrat | – | – | – | – | 0,15 | 0,15 | 0,15 |
| Natrium-metasilikat-pentahydrat | – | – | – | – | 0,10 | 0,10 | 0,10 |
| ε-Aminopentylimidazolin | – | – | – | – | 0,10 | 0,10 | 0,10 |
| Wasser | – | – | – | – | 1,00 | 1,00 | 1,00 |
| Entschäumer auf Silikonbasis | 0,02 | 0,02 | 0,02 | 0,02 | 0,02 | 0,02 | 0,02 |
| Ethylenglykol | 94,98 | 94,98 | 94,03 | 95,58 | 94,48 | 94,48 | – |
| 1,2-Propylenglykol | – | – | – | – | – | – | 94,48 |
| Reservealkalität | 1,75 | 10,20 | 8,40 | 9,70 | 14,10 | 15,50 | 15,60 |

Korrosionsverhalten nach ASTM D 1384–70
Gewichtsabnahme in g/m²

|  | 1 | 2 | 3 | 4 | 5 | 6 | 7 |
|---|---|---|---|---|---|---|---|
| Kupfer | 26,1 | 5,7 | 3,8 | 2,9 | 0,7 | 0,9 | 0,7 |
| Lot auf Messing | 24,9 | 0,2 | 1,9 | 0,7 | 0,9 | 0,0 | 0,1 |
| Messing | 76,0 | 2,8 | 2,4 | 3,1 | 1,5 | 1,6 | 0,9 |
| Stahl | 186,8 | 0,2 | 0,5 | 0,3 | 0,8 | 0,0 | 0,0 |
| Gusseisen | 34,9 | 0,0 | 0,0 | 0,1 | 0,4 | 0,0 | 0,0 |
| Aluminium | 4,8 | 1,7 | 3,5 | 5,8 | 1,5 | 0,9 | 1,2 |

Die Ergebnisse zeigen, dass in bestehenden Rezepturen Natriumbenzoat durch ein Alkalisalz einer Alkyladipinsäure ersetzt werden kann. Das Natriumsalz der Isooctyladipinsäure gibt in jedem Falle erheblich bessere Korrosionsschutzwerte als vergleichsweise Natriumbenzoat oder Borax als alleiniger Inhibitor. In Kombination mit anderen Inhibitoren, beispielsweise nach DE-PS 1 154 976 oder 2 149 138, lässt sich das Natriumsalz der Isooctyladipinsäure auch zur Inhibierung von 1,2-Propylenglykol verwenden. Natriumbenzoat ist in diesem Glykol nicht löslich. (Die obigen Rezepturen sind mit Rücksicht auf einen Einsatz von Alkalisalzen von Alkyladipinsäuren nicht optimiert, d.h. der Zusatz anderer, bekannter Inhibitoren ist hier nicht aufgeführt.)

Beispiel 4

Bremsflüssigkeiten werden auf Korrosion international nach der Spezifikation SAE J 1703f geprüft. Bei diesem Prüfverfahren werden Teststreifen aus den Metallen verzinntes Eisen, Stahl, Aluminium, Guss, Messing und Kupfer nach entsprechender Reinigung in der angegebenen Reihenfolge zusammengeschraubt und in einem Glasbecher mit durchbohrtem Metalldeckel mit der Flüssigkeit überschichtet. Der zu testenden Bremsflüssigkeit werden vorher 5 Gewichtsprozent destilliertes Wasser zugesetzt. Die Prüfung erfolgt 120 Stunden bei einer Temperatur von 100 °C.

Aus den Komponenten Mischaddukt aus 1,5 Mol Propylenoxid an 1 Mol Diethylenglykolmonoethylether, Ethyltriglykol, Butyltriglykol, Diethylenglykol und Polypropylenglykol 620 wurde eine Bremsflüssigkeit mit einem Siedepunkt von 250 °C zusammengestellt. Als Inhibitor wurde einerseits ein Alkaliborat-Glykolkomplex gemäss DE-AS 1 295 124 bzw. DE-OS 1 643 287, anderseits das Kaliumsalz von Isooctyladipinsäuren in etwa gleichen Zusatzmengen verwendet. Der Alkaliborat-Komplexester in Ethylenglykol wurde in einer Zusatzmenge von 3 Gewichtsprozent zugegeben, die Zugabe des Kaliumsalzes erfolgte in einer Menge von 2,5 Gewichtsprozent. Das Kaliumsalz zeigte in der angesetzten Bremsflüssigkeit auch bei tiefen Temperaturen gute Löslichkeit.

Die Hydraulikflüssigkeit aus den vorstehend angegebenen Komponenten hatte einen Siedepunkt nach ASTM D 1120 von 252 °C, eine Viskosität nach ASTM D 445 von 1,7 mm²/s bei 100 °C und 1252 mm²/s bei −40 °C. Nach Zugabe von 3 Gewichtsprozent eines Inhibitorkonzentrates gemäss Beispiel 2 der DE-OS 1 643 287 bzw. 2,5 Gewichtsprozent des trockenen Kaliumsalzes von Isooctyladipinsäuren aus 2,1 Mol Kaliumhydroxid (22,4 g 50%iger, wässriger Kalilauge) und 1 Mol Isooctyladipinsäuren (25,8 g Isooctyladipinsäuren) wurde auch der Oxidationstest nach SAE J 1703f, Section 4, 10, eingehalten. Im Korrosionstest nach SAE J 1703f, Punkt 4,5, wurden folgende Ergebnisse erreicht (Gewichtsverluste in mg/cm²):

|  | Limit nach DOT 3 | mit Inhibitor nach 1 643 287, Beispiel 2 | erfindungsgemäss |
|---|---|---|---|
| verzinntes Eisen | max. $\pm$0,2 | −0,11 | −0,05 |
| Stahl | max. $\pm$0,2 | −0,08 | −0,01 |
| Aluminium | max. $\pm$0,1 | −0,07 | −0,09 |
| Guss | max. $\pm$0,2 | −0,06 | $\pm$0,00 |
| Messing | max. $\pm$0,4 | −0,21 | −0,19 |
| Kupfer | max. $\pm$0,4 | −0,15 | −0,12 |

Der erfindungsgemäss einzusetzende Korrosionsinhibitor zeigt im Vergleich zum Stand der Technik vor allem bei den Eisenmetallen und verzinntem Eisen sehr gute Schutzwerte. Alle Gewichtsverluste entsprechen aber dem Limit nach DOT 3 gemäss Federal Motor Vehicle Safety Standard No. 116, S 5.1.6.

Beispiel 5

Wasserhaltige, nichtentflammbare hydraulische Flüssigkeiten werden heute vor allem in Hydraulikanlagen im Untertagebetrieb im Steinkohlenbergbau verwendet. Die Anforderungen an solche Flüssigkeiten sind in den Berichten der Kommission der europäischen Gemeinschaften festgelegt. Im allgemeinen erfüllen die handelsüblichen Produkte viele der gestellten Bedingungen: gute Feuerbeständigkeit, gewünschter Viskositätsbereich bei hohem Viskositätsindex, Scherbeständigkeit, Korrosionsschutzvermögen, gute Tieftemperatureigenschaften und bergbauhygienische Kriterien. Bis heute unbefriedigend blieb das Schutzvermögen gegenüber Verschleiss.

Solche Flüssigkeiten enthalten mindestens 40 Gewichtsprozent Wasser, 32 bis 40 Gewichtsprozent Glykole und 18 bis 25 Gewichtsprozent eines wasserlöslichen Verdickungsmittels aus einem Mischaddukt aus Ethylenoxid/Propylenoxid im Gewichtsverhältnis 3:1 bis 1,5:1 mit einem Molgewicht von über 5000 und anorganischen und organischen Korrosionsinhibitoren.

Aus folgenden Komponenten wurde eine bekannte Hydraulikflüssigkeit auf Basis einer Polyglykol/Wassermischung hergestellt:

41 Gewichtsprozent destilliertes Wasser
1 Gewichtsprozent Methylmorpholin
0,5 Gewichtsprozent Triethanolamin
0,1 Gewichtsprozent Benzotriazol
37,35 Gewichtsprozent Ethylenglykol
20 Gewichtsprozent eines Mischadduktes aus Ethylenoxid/Propylenoxid vom Molgewicht 12 000
0,05 Gewichtsprozent einer handelsüblichen Silikonemulsion.

Die Ausgangsflüssigkeit hatte eine Viskosität von 38,2 mm²/s (cSt) bei 50 °C, einen Erstarrungspunkt von −41 °C, ein ausreichendes Luftabscheidevermögen nach DIN 51 381 und eine befriedigende, spezifikationsgerechte Scherstabilität nach DIN 51 382.

Dieser Ausgangsflüssigkeit wurden folgende Zusätze zugegeben, um den Reibverschleissschutz zu verbessern:

Stand der Technik 1)
2 Gewichtsprozent Salz der Laurinsäure mit Dibutylamin im Molverhältnis 1:1 (US-PS 2 558 030)

Stand der Technik 2)
3 Gewichtsprozent Ölsäure-Triethanolamin-Salz ebenfalls im Molverhältnis 1:1 (DE-AS 1 804 710)

Tabelle 1

| Verwendete Säure | Stand der Technik | | | | | | | |
| | 1 | 2 | 3 | 4 | 5 | 6 | 7 | 8 |
|---|---|---|---|---|---|---|---|---|
| | Benzoe-säure | 4-tert.-Butyl-benzoesäure | 4-Dodecyl-benzoesäure | Azelainsäure | Sebazinsäure | Adipinsäure | 2,4,4'-Trimethyl-adipinsäure | 2-tert.-Butyladipin-säure |
| Reinheit in Gew.-% | 98 | ca. 95 | ca. 92 | ca. 95 | ca. 98 | 98 | 94 | 92 |
| Molgewicht | 122 | 180 | 290 | 188 | 202 | 146 | 188 | 202 |
| Menge in Gew.-% Triisopropanolamin | 23 | 27 | 32 | 18 | 19 | 15 | 18 | 19 |
| Menge in Gew.-% 1,2-Propylenglykol | 46 | 38 | 31 | 46 | 46 | 50 | 50 | 48 |
| Menge in Gew.-% destilliertes Wasser | 14 | 14 | 14 | 14 | 14 | 12 | 12 | 14 |
| Menge in Gew.-% | 17 | 21 | 23 | 22 | 21 | 23 | 20 | 19 |
| pH-Wert einer 5%igen wässrigen Lösung | 7,45 | 7,4 | 7,5 | 7,45 | 7,4 | 7,55 | 7,4 | 7,45 |

Tabelle 1 (Fortsetzung)

| | Stand der Technik | | | | | | | | | | | | | | | | | | | | | | | |
| | 1 | | | 2 | | | 3 | | | 4 | | | 5 | | | 6 | | | 7 | | | 8 | | |
|---|---|---|---|---|---|---|---|---|---|---|---|---|---|---|---|---|---|---|---|---|---|---|---|---|
| Gew.-% in Trinkwasser 8° dGH | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 |
| Korrosionstest nach DIN 51 360 Teil 1 | 6 | 5 | 3 | 5 | 4 | 2 | 6 | 4 | 1 | 4–5 | 3 | 0 | 5 | 2–3 | 1 | 6 | 6 | 5 | 6 | 6 | 6 | 5 | 4 | 3 |
| Korrosionstest nach DIN 51 360 Teil 2 (Span-Test) | 4 | 4 | 3–4 | 4 | 4 | 3 | 4 | 2 | 1–2 | 4 | 2–3 | 0–1 | 4 | 3 | 1 | 4 | 4 | 4 | 4 | 4 | 4 | 4 | 4 | 3–4 |

0 029 892

Tabelle 2

| | erfindungsgemäss | | | | Stand der Technik | | |
|---|---|---|---|---|---|---|---|
| | 9 | 10 | 11 | 12 | 13 | 14 | 15 |
| Verwendete Säure oder Inhibitor | Hexyla-dipin-säure | Isooctyl-adipin-säure | Isononyl-adipin-säure | Dodecyl-adipin-säure | Ölsäure | Handels-produkt A[1] | Handels-produkt B[2] |
| Reinheit in Gew.-% | ca. 93 | ca. 94 | ca. 92 | ca. 93 | ca. 96 | – | – |
| Molgewicht | 230 | 258 | 272 | 300 | 282 | – | – |
| Menge in Gew.-% Triisopropanolamin | 25 | 24 | 25 | 27 | 26 | 100 | 70 |
| Menge in Gew.-% 1,2-Propylenglykol | 49 | 46 | 45 | 44 | 46 | – | – |
| Menge in Gew.-% destilliertes Wasser | 12 | 14 | 14 | 14 | 14 | – | 14 |
| Menge in Gew.-% | 15 | 16 | 16 | 15 | 14 | – | 16 |
| pH-Wert einer 5%igen wässrigen Lösung | 7,55 | 7,55 | 7,6 | 7,6 | 7,65 | 8,35 | 10,3 |

[1] Salz einer Arylsulfonamidoalkylencarbonsäure mit Triethanolamin gemäss DE-PS 1 298 672
[2] Mischung aus Fettsäurediethanolamid mit Fettsäurediethanolaminsalzen

Tabelle 2 (Fortsetzung)

| | erfindungsgemäss | | | | | | | | | | | | Stand der Technik | | | | | | | | |
|---|---|---|---|---|---|---|---|---|---|---|---|---|---|---|---|---|---|---|---|---|---|
| | 9 | | | 10 | | | 11 | | | 12 | | | 13 | | | 14 | | | 15 | | |
| Gew.-% in Trink-wasser 8° dGH | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 |
| Korrosionstest nach DIN 51 360 Teil 1 | 3 | 0 | 0 | 2–3 | 0 | 0 | 3 | 0–1 | 0 | 3 | 0 | 0 | 6 | 4 | 2 | 4 | 2 | 0 | 5 | 3–4 | 1–2 |
| Korrosionstest nach DIN 51 360 Teil 2 (Span-Test) | 3–4 | 1–2 | 0 | 2–3 | 1 | 0 | 3–4 | 1 | 0 | 3–4 | 2 | 0 | 4 | 4 | 3–4 | 4 | 3–4 | 0–1 | 4 | 3–4 | 2–3 |

Tabelle 3

0 029 892

| | 16 | | | 17 | | | 18 | | | 19 | | | 20 | | | 21 | | | 22 | | | 23 | | |
|---|---|---|---|---|---|---|---|---|---|---|---|---|---|---|---|---|---|---|---|---|---|---|---|---|
| Verwendete Säure | Isooctyl-adipinsäure | | | Isooctyl-adipinsäure | | | Isooctyl-adipinsäure | | | Isononyl-adipinsäure | | | Isononyl-adipinsäure | | | Isododecyl-adipinsäure | | | Azelainsäure | | | Sebazin-säure | | |
| Molgewicht | 258 | | | 258 | | | 258 | | | 272 | | | 272 | | | 300 | | | 188 | | | 202 | | |
| Alkali | Natrium | | | Kalium | | | Kalium | | | Natrium | | | Kalium | | | Natrium | | | Natrium | | | Natrium | | |
| Alkaligehalt in Mol | 2,1:1 | | | 2,1:1 | | | 2:1 | | | 2:1 | | | 2,1:1 | | | 2:1 | | | 2,1:1 | | | 2:1 | | |
| 1,2-Propylenglykol Menge in Gew.-% | – | | | – | | | 54 | | | – | | | – | | | – | | | – | | | – | | |
| Wasser, Menge in Gew.-% | – | | | – | | | 6 | | | – | | | – | | | – | | | – | | | – | | |
| pH-Wert einer 5%igen wässrigen Lösung | 7,3 | | | 8,0 | | | 7,8 | | | 7,6 | | | 7,8 | | | 8,0 | | | 7,9 | | | 7,9 | | |
| Gew.-% in Trinkwasser 8° dGH | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 | 0,5 | 1,0 | 2,0 |
| Korrosionstest nach DIN 51 360 Teil 1 | 2 | 0 | 0 | 1 | 0 | 0 | 2–3 | 1 | 0 | 1–2 | 0 | 0 | 1 | 0 | 0 | 1–2 | 0 | 0 | 2 | 1 | 0 | 3 | 1–2 | 0 |
| Korrosionstest nach DIN 51 360 Teil 2 (Span-Test) | 2 | 0 | 0 | 1–2 | 0–1 | 0 | 2 | 1 | 0 | 0–1 | 1 | 0 | 1 | 0 | 0 | 1 | 0 | 0 | 3 | 1–2 | 0 | 4 | 2 | 0 |

Stand der Technik 3)

3 Gewichtsprozent eines Kaliumsalzes der Sebazinsäure eines pH-Wertes einer 5%igen, wässrigen Lösung von 7,6 (nach US-PS 2 737 497)

Stand der Technik 4)

0,5 Gewichtsprozent eines Diethanolaminsalzes der Dodecylbernsteinsäure (nach DE-AS 1 594 570)

Erfindungsgemäss 5)

3 Gewichtsprozent eines Diethanolaminsalzes von Isododecyladipinsäuren

Auf dem Vierkugelapparat nach Boerlage gemäss DIN 51 350 wurde je ein Versuch von 1 Stunde Dauer mit einer Belastung von 60 kg durchgeführt. Dabei wurden folgende Verschleiss-Kalottendurchmesser in mm festgestellt:

Ausgangsflüssigkeit　0,26　Stand der Technik
Zusatz 1　　　　　　　0,19　Stand der Technik
Zusatz 2　　　　　　　0,18　Stand der Technik
Zusatz 3　　　　　　　0,20　Stand der Technik
Zusatz 4　　　　　　　0,23　Stand der Technik
Zusatz 5　　　　　　　0,14　Erfindungsgemäss

Durch den erfindungsgemässen Zusatz 5 konnte eine deutliche Verbesserung des Reibverschleissverhaltens erreicht werden. Die angegebenen Messdaten sind Mittelwerte aus 6 Versuchen.

**Patentansprüche**

1. Verwendung von Dialkalisalzen oder Disalzen wasserlöslicher aliphatischer Amine eines Gemisches aus 2- und 3-Alkyladipinsäuren, deren Alkylrest verzweigt oder unverzweigt ist und 6 bis 12 Kohlenstoffatome enthält, als Korrosionsinhibitor für Bohr-, Schneid- und Schleifmittel, Hydraulikflüssigkeiten und Gefrierschutzmittel, welche Wasser als Hauptbestandteil enthalten.

2. Verwendung nach Anspruch 1, dadurch gekennzeichnet, dass die Alkali- oder Aminsalze in Konzentrationen von 0,5 bis 3,0 Gewichtsprozent, bezogen auf das Bohr-, Schneid- oder Schleifmittel, eingesetzt werden.

3. Verwendung nach Anspruch 1, dadurch gekennzeichnet, dass die Alkali- oder Aminsalze in Konzentrationen von 1 bis 5 Gewichtsprozent, bezogen auf die Hydraulikflüssigkeit, eingesetzt werden.

4. Verwendung nach Anspruch 1, dadurch gekennzeichnet, dass die Alkali- oder Aminsalze in Konzentrationen von 0,6 bis 1,7 Gewichtsprozent, bezogen auf das gesamte Gefrierschutzmittel, eingesetzt werden.

5. Verwendung nach Anspruch 1, dadurch gekennzeichnet, dass die Alkali- oder Aminsalze gemeinsam mit üblichen Korrosionsinhibitoren in Konzentrationen von 0,6 bis 1 Gewichtsprozent, bezogen auf das gesamte Gefrierschutzmittel, eingesetzt werden.

**Claims**

1. The use of a di-alkali metal salt, or of a disalt of a water-soluble aliphatic amine, of a mixture of 2-alkyl- and 3-alkyl-adipic acids whose alkyl radicals are branched or straight-chain and have 6 to 12 carbon atoms as corrosion inhibitor for boring, cutting and grinding fluids, hydraulic fluids and antifreezes which contain water as a substantial ingredient.

2. A use according to claim 1, characterised in that the alkali metal or amine salt is used in a boring, cutting or grinding fluid in a concentration of 0.5 to 3.0% by weight based on the fluid.

3. A use according to claim 1, characterised in that the alkali metal or amine salt is used in a hydraulic fluid in a concentration of 1 to 5% by weight based on the fluid.

4. A use according to claim 1, characterised in that the alkali metal or amine salt is used in an antifreeze in a concentration of 0.6 to 1.7% by weight based on the whole antifreeze.

5. A use according to claim 1, characterised in that the alkali metal or amine salt is used together with a conventional corrosion inhibitor in an antifreeze in a concentration of the alkali metal or amine salt of 0.6 to 1% by weight based on the whole antifreeze.

**Revendications**

1. Utilisation de sels dialcalins ou de sels d'amines aliphatiques hydrosolubles d'un mélange d'acides 2- et 3-alkyl-adipidiques dont le radical alkyle est ramifié ou non ramifié et contient de 6 à 12 atomes de carbone, comme inhibiteur de corrosion pour agents de perçage, de coupe et de meulage, liquides hydrauliques et antigels qui contiennent de l'eau comme constituant principal.

2. Utilisation selon la revendication 1, caractérisée par le fait que l'on utilise les sels alcalins ou d'amines à des concentrations de 0,5 à 3,0% en poids, relativement à l'agent de perçage, de coupe ou de meulage.

3. Utilisation selon la revendication 1, caractérisée par le fait que l'on utilise les sels alcalins ou d'amines à des concentrations de 1 à 5% en poids, relativement au liquide hydraulique.

4. Utilisation selon la revendication 1, caractérisée par le fait que l'on utilise les sels alcalins ou d'amines à des concentrations de 0,6 à 1,7% en poids, relativement au total de l'antigel.

5. Utilisation selon la revendication 1, caractérisée par le fait que l'on utilise les sels alcalins ou d'amines conjointement avec des inhibiteurs de corrosion usuels, à des concentrations de 0,6 à 1% en poids, relativement au total de l'antigel.